# EUROPÄISCHE PATENTSCHRIFT

(11) **EP 3 986 347 B1**
(45) Veröffentlichungstag und Bekanntmachung des Hinweises auf die Patenterteilung: **22.01.2025**
(21) Anmeldenummer: 20734685.9
(22) Anmeldetag: 22.06.2020
(51) Int. Cl.: A61F 13/01, A61L 15/26, A61F 13/02, A61F 13/06, A61F 13/08, A61L 15/42, D04B 21/16, D04B 21/18, D04H 1/52

(54) **KOMPRESSIONSBINDE SOWIE KOMPRESSIONSBINDENZUSAMMENSTELLUNG**
COMPRESSION BANDAGE AND COMPRESSION BANDAGE COMBINATION
BANDE DE COMPRESSION AINSI QU'ENSEMBLE DE BANDE DE COMPRESSION

(30) Priorität: 21.06.2019 DE 102019116825
(43) Veröffentlichungstag der Anmeldung: 27.04.2022
(73) Patentinhaber: KOB GmbH, 67752 Wolfstein (DE)
(72) Erfinder: TAMOUÉ, Ferdinand, 67071 Ludwigshafen am Rhein (DE); DR. LANGEN, Günter, 67752 Wolfstein (DE)
(74) Vertreter: DREISS Patentanwälte PartG mbB
(86) Internationale Anmeldenummer: PCT/EP2020/067267
(87) Internationale Veröffentlichungsnummer: WO 2020/254676

(56) Entgegenhaltungen:
- DE-A1- 102015 226 645
- DE-A1- 102015 226 706
- DE-A1- 19 819 442

## Beschreibung

Die Erfindung betrifft eine Kompressionsbinde sowie eine Kompressionsbindenzusammenstellung umfassend zwei Schichten, sowie Verfahren zur Herstellung von Kompressionsbinden und deren Verwendung.

Kompressionsbinden werden im Stand der Technik beispielsweise für diabetische Ulzera eingesetzt. Hierbei besteht das Problem, dass aufgrund des Krankheitsbildes notwendigerweise verschiedene Fragestellungen zu berücksichtigen sind, nämlich zum einen eine hinreichende Kompression, zum anderen aber auch eine Polsterwirkung gegenüber der zu behandelnden Gliedmaße.

So kann es beispielsweise vorgesehen sein, eine Polsterung aufzubringen und darüber eine Kompressionsbinde anzulegen. Darüber hinaus sieht die Lehrmeinung jedoch eine Kontraindikation bei gleichzeitiger arterieller Verschlusskrankheit.

Dabei sind bei der Kompressionstherapie grundsätzlich der sogenannte Arbeitsdruck und der sogenannte Ruhedruck zu unterscheiden, wobei der Ruhedruck der Druck ist, der bei liegender Gliedmaße durch das Kompressionsmittel, hier den Kompressionsverband, auf die Gliedmaße ausgeübt wird. Der Arbeitsdruck ist dann der Druck, der auf die Gliedmaße bei Bewegung der Muskeln ausgeübt wird. Bevorzugt soll der Arbeitsdruck 20 bis 40 mm Hg über dem Ruhedruck liegen.

Es ist dabei ebenfalls bekannt, verschiedene Bindentypen einzusetzen. So sind sogenannte Langzugbinden bekannt, die eine sehr hohe Elastizität aufweisen und häufig eine Dehnfähigkeit über 200 % besitzen, wobei demgegenüber Kurzzugbinden bekannt sind, die nur eine geringe Dehnbarkeit besitzen und nur eine geringe Rückstellkraft, jedoch bereits sehr früh keine weitere Dehnung zulassen und so einen vergleichsweise hohen Arbeitsdruck aufzubauen vermögen. Demgegenüber bauen Kurzzugbinden über einen vergleichsweise langen Bereich nur einen geringen Widerstand auf, um dann eine sehr hohe Begrenzung der Dehnbarkeit zu realisieren. Die traditionelle Kurzzugbandagenkompressionstherapie ist eine Maßnahme für die Behandlung von Venenerkrankungen. Dabei werden die Materialien für Kurzzugbinden in der Regel aus nicht elastischen Materialien gefertigt und durch Ausrüstungsprozesse elastifiziert. Die Elastizität reduziert sich jedoch während der Behandlung signifikant. Dies kann zu einer Reduktion des Kompressionsdrucks in der Anwendung führen.

Bekannte Kompressionsbandagen sind beispielsweise in der EP 2 275 062 A2 beschrieben, die eine innere der Haut zugewandte elastische Bandage beschreibt mit einem gedehnten elastischen Substrat und einer gedehnten Schaumschicht, die auf der hautzugewandten Seite des Substrats angeordnet ist, sowie einer weiteren gedehnten selbstklebenden elastischen Bandage, die hierüber angelegt wird.

Darüber hinaus ist ein Stütz-, Fixier- oder Druckverband aus der DE 20 2012 000 529 U1 bekannt, wobei der Verband hier mindestens vier Lagen aufweist und eine Spannlage besitzt, die die Rückstellkraft erzeugt und Löcher aufweist, wobei die übrigen Lagen durch die Löcher miteinander fixiert sind. Durch die vier vorgesehenen Lagen ist der Verband vergleichsweise teuer und aufwendig.

Des Weiteren ist noch auf die WO 2014/131976 A2 hinzuweisen, die ebenfalls eine elastische Bandage betrifft, umfassend eine nicht elastische Schicht, die die elastische Bande umschließt und hiermit verbunden ist.

Aus der DE 10 2015 226 706 A1 ist ein Kompressionsverband bekannt, mit einer Polsterschicht und einer Stützschicht, die mittels Nähwirkverfahren miteinander verbunden sind, wobei hierüber ein zweiter Kompressionsverband anlegbar ist.

Weitere Kompressionsbinden sind aus DE19819442 und DE102015226645 bekannt.

Es ist wünschenswert, eine Kompressionsbinde, die alternativ auch als Kompressionsverband oder -bandage bezeichnet werden kann, bereitzustellen sowie eine Kompressionsbindenzusammenstellung, die eine hohe Anlegesicherheit aufweisen bei gleichzeitig guten therapeutischen Eigenschaften.

Sofern die Begriffe Schicht oder Lage verwendet werden, bezeichnen diese den gleichen Gegenstand.

Ausgehend von diesem Stand der Technik löst die Erfindung die Aufgabe durch eine Kompressionsbinde mit den Merkmalen des Anspruchs 1 sowie eine Kompressionsbindenzusammenstellung des Anspruchs 8, sowie einem Verfahren zum Herstellen einer Kompressionsbinde des Anspruchs 14 und einer Kompressionsbinde zur Verwendung nach Anspruch

Die Kompressionsbinde umfasst eine vliesbasierte Bindenlage, die durch elastische Textilfäden mittels Nähwirkverfahren übernäht ist, wobei die Textilfäden hitzeschrumpffähig und nicht elastomer sind und die Kompressionsbinde eine elastische Dehnbarkeit nach Hitzeschrumpfung der Textilfäden um 50-200% aufweist, vorzugsweise kann die Hitzeschrumpfbarkeit der Textilfäden 50-90% und weiter vorzugsweise kann die Hitzeschrumpfbarkeit 50 bis 70% betragen. Durch die Hitzebehandlung erfahren die hitzeschrumpffähigen Textilfäden, die in das Vlies mittels des Nähwirkverfahrens verarbeitet wurden, einen Schrumpf und verkürzen sich. Dadurch verkürzt sich auch die gesamte Vlieskonstruktion und erhält dadurch ihre Rückstellfähigkeit und dehnungselastischen Eigenschaften. Die Schrumpfung tritt dabei vorzugsweise in Längsrichtung auf. Die Hitzeschrumpfbehandlung des Nähwirkvlies erfolgt vorzugsweise mit Heißluft (130 °C bis 200 °C) oder Wasserdampf (Sattdampf oder gespannter Wasserdampf mit 100°C bis 140 °C).

Bei der Kompressionsbinde handelt es sich vorzugsweise um eine Binde mit Rückzugswerten kleiner 90 % (DIN 61632). Nach einem ersten Ausführungsbeispiel kann die Bindenlage aus einem chemisch, thermisch und/oder mechanisch verfestigten Vliesstoff bestehen und insbesondere ein Thermobondvlies sein.

Bei dem Grundvlies kann es sich vorzugsweise um ein Faservlies (Stapelvlies) aus Baumwolle, Wolle, Viskose, Polyamid, Polyester, Acryl, Polyolefin oder Mischungen dieser Faserstoffe handeln, insbesondere kann es hieraus gebildet sein, wobei diese Vliese insbesondere mechanisch oder chemisch gebunden sind. Alternativ kann ein Spunbond aus Polyamid, Polyester, Polyolefin, Acryl oder Mischungen hiervon bevorzugt eingesetzt werden.

Bei den hitzeschrumpffähigen Textilfäden kann es sich vorzugsweise um texturierte Multifilament-Garne, Bikomponenten-Polymerfasern und Microfasern handeln. Insbesondere kann vorgesehen sein, dass die Textilfäden texturierte Polyamidgarne und/oder texturierte Polyestergarne sind. Dabei kann insbesondere vorgesehen sein, dass es sich bei den Nähfäden um 78 dtex bis 320 dtex texturiertes Multifilamentgarn aus Polyamid, Polyester, Polyethylenepthalat oder Polybutylenephthalat oder Mischungen hieraus handelt. Insbesondere kann Polyamid 6 bis 6.6 eingesetzt werden.

Es kann vorgesehen sein, dass der Vliesstoff an sich, ohne die hitzeschrumpffähigen Textilfäden unelastisch oder dehnungselastisch ist.

Die Kompressionsbinde kann vorzugsweise kohäsiv ausgebildet sein.

Als Flächengewichte der Bindenlage kommen 20 bis 40 g/m², wobei sowohl weiße als auch farbige Bindenlage eingesetzt werden können.

Die Bindenlage kann dabei glatt oder offenporig oder perforiert oder geprägt sein.

Zum Übernähen wird eine Nähfadendichte von 74 bis 96 Fäden je cm Bindenbreite eingesetzt. Als bevorzugte Nähfadenstichlänge sind 2 bis 5 mm anzusehen.

Für die Nähfadenbinden kommen sowohl offene als auch geschlossene Fransen oder Trikotbindungen oder Kombinationen hieraus in Frage. Die Kompressionsbinde weist ein Flächengewicht von 25 bis 28 g/m² (gedehnt) ohne kohäsive Beschichtung und von 30 bis 100 g/m² (gedehnt) in der kohäsiv beschichteten Form auf.

Als Dehnbarkeit nach DIN 61632 bei einer Kraft von 3 N/cm ergibt sich eine Dehnbarkeit von 40 bis 100% in Längsrichtung (MD) sowie 0 bis 50% in Querrichtung (CD) Die Erfindung umfasst ebenfalls eine Kompressionsbindenzusammenstellung gemäß Anspruch 8 umfassend eine erste innere Binde sowie eine zweite äußere Binde, wobei die zweite äußere Binde im Gebrauch über die erste innere Binde anlegbar ist.

Derartige mehrlagige Verbände (Multilayerverband) zur Verwendung als Kompressionsverband am menschlichen oder tierischen Körper sind bekannt. Die Verbände werden dabei durch Übereinanderwickeln von mindestens zwei separaten unterschiedlichen Binden am Körperteil angefertigt. Dabei ist es bekannt, die innere Binde als Polsterbinde zu gestalten, die direkt auf die Haut des Körperteils als innere Lage angewickelt wird und darüber eine Kompressionsbinde zu platzieren, die im Anschluss an die erste innere Binde als äußere Lage über diese gewickelt wird, wobei beide Lagen aufeinander haften und eine rutschsichere Verbindung eingehen.

Dabei umfasst die erste innere Binde einen vorzugsweise ersten Abschnitt (L1), der zumindest auf seiner der Haut eines Trägers zugewandten Seite eine Polsterschicht aufweist und insbesondere einen kohäsiv haftenden zweiten Abschnitt (L2) aufweist und wobei die zweite äußere Binde eine Kompressionsbinde der vorstehend beschriebenen Art ist. Bei der Kompressionsbindenzusammenstellung handelt es sich insbesondere um einen Zweilagen-Kompressionsverband mit fixem Kompressionsdruck umfassend eine erste Binde (Komponente A: Polsterbinde) und eine zweite Binde (Komponente B: Kompressionsbinde gemäß der Erfindung), wobei zuerst die Komponente A am Körperteil angelegt wird und dann darüber die Komponente B gewickelt wird und diese Kombination aus Lage A und B einen fixen Kompressionsdruck auf das Körperteil ausübt. Insbesondere umfasst die vorliegende Erfindung eine Kompressionsbinde zur Behandlung venöser Erkrankungen wie der chronischen venösen Insuffizienz oder chronischer venöser Ulcera, aber auch Erkrankungen mit arterieller Beteiligung wie der peripheren arteriellen Verschlusskrankheit (pAVK) oder sog. gemischte Ulcera.

Die Erfindung löst hierdurch das weitere technische Problem eines medizinischen Mehrkomponenten-Kompressionsverbands aus zwei getrennten elastischen Kurzzugsbinden mit limitiertem und definiertem therapeutischen Druck. Die Kombination der Komponenten ermöglicht aufgrund der geringeren Rückstellkraft und dem deshalb dabei limitierten Anpressdruck die Herstellung eines Ausgangmaterials, dessen primäre Funktion eine Kompressionsbinde für die Behandlung der peripheren arteriellen Verschlusskrankheit ist.

Die Kompressionstherapie der Unterschenkel ist eine der wesentlichen Therapiepfeiler zur Behandlung der chronischen venösen Insuffizienz (CVI) bis hin zum Ulcus cruris venosum (UCV). In vielen Übersichtsarbeiten und Leitlinien wird der Effektivität dieser Therapie eine Level-1-Evidenz zur Ulkus-Abheilung und auch zur Rezidivprophylaxe bescheinigt. Gleichzeitig findet sich in dieser Literatur aber auch der Hinweise, die periphere arterielle Verschlusskrankheit (pAVK) als relative Kontraindikation und die fortgeschrittene pAVK als absolute Kontraindikation zu beachten. Einige Autoren konkretisieren diese Angaben mit einer absoluten Kontraindikation bei einem Dopplerindex (ABI) von <0,6 oder auch schon bei <0,8. Anwendungsbeobachtungen zeigen jedoch für eine moderate Kompression (30 mmHg) bei Patienten mit Ulcus cruris und pAVK (ABI 0,5-0,8) über 14 Tage keine Komplikationen und eine gute Verträglichkeit. Bei einer CVI mit Ulcus cruris und gleichzeitig diagnostizierter pAVK (ABI 0,5-0,8) wird die arterielle Perfusion durch die angewandte Kurzzugkompression nicht eingeschränkt, die reduzierte venöse Pumpfunktion vor allem beim Gehen aber verbessert. Die Therapie des UCV mit einer Kompression <40 mmHg ist trotz einer vorhandenen pAVK (ABI 0,5-0,8) problemlos möglich, die Abheilung allerdings verzögert. Bei arteriovenösen Ulzera (ABI >0,6) führt die Kurzzugkompression bis 40 mmHg zu einer Verbesserung des arteriellen Flusses und der venösen Pumpfunktion. Zusammenfassend scheint eine Kompressionstherapie auch bei einer begleitenden pAVK bis zu einem (Grenz-) Wert-ABI von circa 0,5 problemlos möglich. Eine erfindungsgemäße Kompressionsbinde sowie Kompressionsbindenzusammenstellung ist in der Lage, einen maximalen Kompressionsdruck von weniger als 40 mmHg zu garantieren, selbst bei maximaler Dehnung.

Die Erfindung umfasst also auch die Verwendung einer erfindungsgemäßen Kompressionsbinde nach Anspruch 15 zur Behandlung von Venenerkrankungen, chronischer venöser Insuffizienz, Ulcus cruris venosum auch bei begleitender peripherer arterieller Verschlusskrankheit.

Es ist dabei vorgesehen, dass die beiden Abschnitte (L1, L2) der ersten inneren Binde miteinander verbunden sind und in Längsrichtung der Binde aneinander anschließen oder einander ganz oder teilweise überdecken.

Ferner kann vorgesehen sein, dass die erste Binde eine erste Polsterschicht und eine zweite Stützschicht umfasst und die beiden Schichten im ungedehnten Zustand mittels Nähwirkverfahren über einen elastischen Nähfaden miteinander verbunden sind und die Stichläge 1,5 bis 3 mm/U beträgt bei einer Nähfadenspannung von höchstens 4 cN. Die Elastizität kann dabei durch die Einarbeitung von elastischen Fäden, vorzugsweise Gummi- oder Polyurethanfäden eingestellt werden. So kann z.B. die Elastizität durch Übernähen eines starren einlagigen Vliesstoffes mit dauerelastischen Elastanfäden in Längsrichtung unter Anwendung der Nähwirktechnik MALIWATT erhalten werden. Die Nähwirktechnik MALIWATT ist in Malimo Nähwirktechnologie, Ploch, Böttcher, Scharch, VEB Fachbuchverlag Leipzig, 1978, 1. Auflage beschrieben. Mit der gleichen Technik können auch 2 übereinandergestapelte Vliesstoffflächen (Flächengebilde 1: Standardvlies, Flächengebilde 2 flauschiges Wattevlies) zu einer elastischen Verbund- Vliesfläche mit Flauschstruktur vernäht werden.

Der Vorteil bei einem Nähwirkverfahren besteht darin, dass gleichzeitig an mehreren Stellen eine Verbindung durchgeführt werden kann und die beiden Schichten nach der Verbindung nicht mehr voneinander getrennt werden können. Über die Auswahl der Stichlänge in Längsrichtung des Flächengebildes aus dem dann die Kompressionsbinde oder die innere Binde konfektioniert werden, wobei unter Stichlänge der Abstand in Stichlängsrichtung zwischen zwei Stichen verstanden werden soll, und der Nähfadenspannung die Dehnbarkeit des elastischen Verbundes eingestellt werden kann, so dass ein von Hand nicht mehr trennbarer und trotzdem kontrollierbar elastischer Verbund aus den beiden Schichten besteht. Je nach Auswahl dieser Parameter zieht sich das fertige Flächengebilde zusammen bei Entspannung und werden Falten im Material aufgeworfen.

Die Nähtechnik und die Stichlänge des Nähfadens für die innere Binde werden dabei so reguliert, dass die Fasern auf der Polsterschicht auf der einen Seite des Verbundes aus den zwei Schichten Hautkomfort und Ausgleichsfunktionen besitzen und damit letztendlich die innere Binde zwei erkennbar unterschiedliche Seiten aufweist, die für den Druckausgleich sehr funktional sind. Des Weiteren ist die Stichlänge dabei so einzustellen, dass die gewünschten absorbierenden sowie hautfreundlichen Eigenschaften der der Haut zugewandten Polsterschicht erhalten bleiben.

Die Polsterschicht ist dabei die einer Gliedmaße zugewandte Seite der inneren Binde und die Stützschicht die hierauf aufgebrachte zweite Seite.

Das Malimo- bzw. Maliwattverfahren zum Verbinden der Schichten der inneren Binde bzw. zur Herstellung der inneren Binde oder der Kompressionsbinde wird dabei, wie im Stand der Technik bekannt eingesetzt. So kann z.B. die Elastizität durch Übernähen eines starren Vliessstoffes oder Gewebes mit dauerelastischen Elastanfäden in Längsrichtung unter Anwendung der Nähwirktechnik MALIWATT oder Malimo erhalten werden. Die Nähwirktechnik MALIWATT bzw. Malimo ist in Malimo Nähwirktechnologie, Ploch, Böttcher, Scharch, VEB Fachbuchverlag Leipzig, 1978, 1. Auflage beschrieben.

Das Material der inneren Binde und/oder der äußeren Binde sowie einer oder beider der Binden-Schichten (sofern die Binden mehrschichtig sind) selber kann unter anderem unelastisch sein. Hierdurch kann die Elastizität, die dann durch das Übernähen bereitgestellt wird, besonderes präzise eingestellt werden. Die Elastizität kann in Längs- und/oder Querrichtung vorliegen, vorzugsweise in Bindenlängsrichtung.

Durch diese Gestaltung kann bei mehrschichtigen Binden ferner erreicht werden, dass die beiden Schichten mittels des Nähwirkverfahrens und hier vorzugsweise einem Malimo- bzw. Maliwatt-Verfahren verbunden werden, wobei die Schichten im ungedehnten Zustand mittels des elastischen Nähfadens verbunden werden.

Bevorzugt können die Kompressionsbinde (äußere Binde) und/oder die innere Binde, und, sofern vorgesehen, beide Schichten der inneren Binde, also die Polsterschicht und die Stützschicht unelastisch ausgebildet sein und werden erst durch das Nähwirkverfahren elastifiziert.

Weiterhin ist für die innere Binde vorgesehen, dass mindestens eine der beiden Schichten aus Polsterschicht und Stützschicht aus einem Vliesmaterial bestehen. Dabei kann insbesondere vorgesehen sein, dass das Flächengebilde mit Flauschstruktur aus einem ein- oder mehrlagigen watteähnlichem Vliesstoff besteht.

Alternativ sind jedoch auch andere Materialien für die innere Binde und insbesondere eine oder beide Schichten der inneren Binde einsetzbar, wie z.B. Gewebe, Gewirke, Gestricke oder Schäume einsetzbar. Dabei kann es sich z. B. bei der Polsterschicht um eine Wattevliesschicht, insbesondere eine Thermofusionsvliesschicht, handeln, die gegebenenfalls auch schon vorvernadelt sein kann.

Dabei werden bei beiden Verfahren, nämlich dem Thermobonding als auch der der Thermofusion die Fasern der Vliese in einem Kämmereiverfahren in eine bestimmte Richtung gelegt und in einem Textilfunktionalisierungsverfahren als Vliesrollen vorbereitet und durch Temperatur oder durch Temperatur und Druck stabilisiert für die Weiterverarbeitung. Während des Thermofusionsverfahrens werden Fasern mit verschiedenen Schmelzpunkten durch Heißluft-Trockner miteinander verschmolzen. Im Thermobondingverfahren werden die Fasern mittels Hitze und Druck zwischen beheizten Kalanderrollen verschmolzen. Das Ergebnis sind in beiden Fällen weiche, homogene Vliesstoffe, die ideal und für technische Anwendungen geeignet sind. Das Thermofusionsverfahren eignet sich aufgrund des fehlenden Drucks besser für Polsterschichten.

Um eine optimale Polsterwirkung zu erzielen, kann es bevorzugt vorgesehen sein, dass die Dicke der Polsterschicht der inneren Binde 0,3-12 mm, bevorzugt 0,4-6 mm und weiter bevorzugt 0,5-3 mm, besonders bevorzugt 0,6-1,2mm beträgt.

Bei der Stützschicht der inneren Binde kann es sich um ein Thermobondvlies handeln. Das Thermobondvlies weist dabei vorzugsweise lediglich eine geringe Dehnfähigkeit bei gleichzeitig gewünschter Steifigkeit auf.

Ebenfalls ist das Vliesmaterial der Kompressionsbinde (äußere Binde) vorzugsweise ein Thermobondvlies. Zum Übernähen wird das Vliesmaterial der jeweiligen Binden einer Kettenwirkmaschine zugeführt und mittels eines elastischen Nähfadens, der vorzugsweise aus einer Gruppe aus Baumwollspinnkreppfäden, Baumwollzwirnkreppfäden, texturierten Polyamidgarnen, texturierten Polyestergarnen, Gummifäden oder Polyurethanelastanfäden oder einer Kombination hiervon ausgewählt werden kann, übernäht und bei mehreren Schichten miteinander verbunden. Bei dem Material zur Herstellung der Kompressionsbinde handelt es sich um nichtelastische Thermoplasten, die normalerweise nicht geeignet sind, zur Herstellung von elastischen Flächengebilden, da sie durch ihr Spinnverfahren eher eine höhere kristalline Struktur, verglichen mit Elastomeren, haben. Durch den Mangel an amorpher Struktur, welcher im Gegenteil dazu bei Elastomeren für eine gute Dehnbarkeit sorgt.

Der Nähfaden kann alternativ auch als Kettfaden bezeichnet sein. Der Faden verläuft dabei in Maschinenrichtung der Kettenwirkmaschine und nicht quer hierzu.

Das fertig vernähte Flächengebilde der inneren Binde und/oder der Kompressionsbinde weist stets eine optimierte Dehnung auf, wobei besonders bevorzugt vorgesehen sein kann, dass die maximale Dehnbarkeit der inneren Binde und/oder der Kompressionsbinde, der einer vorgegebenen optimalen Dehnbarkeit entspricht, und eine hierüber hinausgehende Dehnung durch eine Dehnungsschwelle begrenzt ist. Auf diese Weise kann die Anlegesicherheit signifikant erhöht werden, da es auch für nicht geübte Anwender möglich ist, die innere Binde und/oder die Kompressionsbinde maximal bis zur Dehnungsschwelle zu dehnen, wobei dann nicht nur die maximale Dehnbarkeit, sondern zugleich auch die optimale Dehnung und damit der optimale Kompressionsdruck erreicht ist und in diesem maximal gedehnten Zustand die innere Binde und/oder die Kompressionsbinde anzulegen.

Aufgrund des Übernähens mittels eines Nähwirkverfahrens werden die innere Binde und/oder die Kompressionsbinde im entspannten Zustand, nachdem das vernähte Flächengebilde mittels Längskonfektionierung weiterverarbeitet wurde, so in Wellen gelegt, dass eine unregelmäßige Oberfläche entsteht. Aufgrund dieser unregelmäßigen Oberfläche wird neben der primären Funktion als Ausgleichslage und der sekundären Funktion der regulierbaren Dehnbarkeit und damit erhöhten Anlegesicherheit auch erreicht, dass durch die Wellen ein Muster auf der Oberfläche entsteht aus Materialerhebungen und Materialvertiefungen, dass auch im maximal gedehnten Zustand nicht vollständig aufgehoben ist, so dass in der Therapie hierdurch zusätzlich ein Massage- bzw. Drainageeffekt entsteht.

Die Einteilung in die Kategorien Kurz-, Mittel- oder Langzugbinde erfolgt je nach Dehnbarkeit und kann z.B. P. Asmussen, B. Söllner, Kompressionstherapie Prinzipien und Praxis, Verlag Urban & Fischer in Elsevier, 2004 auf Seite 121 entnommen werden. Die Dehnbarkeiten werden dabei nach DIN 61632 bestimmt.

Weiterhin kann vorgesehen sein, dass sich eine Dehnbarkeit der Kompressionsbinde (zweiten äußeren Binde) gemessen nach DIN 61632 von D_{fix} >90%, insbesondere von 40%- 80% ergibt.

Beim Anlegen der Kompressionsbindenzusammenstellung ist es vorteilhaft, wenn die erste und die zweite Binde sich vollflächig und insbesondere kantengenau überdecken und über den gesamten Dehnungsbereich 0 bis Dfix haftend miteinander verbunden sind.

Vorzugsweise sind beide Binden kohäsiv haftend ausgebildet. Es kann dann eine rutschsichere Verbindung aufgrund des kohäsiv haftenden zweiten Abschnitts der inneren Binde und der hiermit zusammenwirkenden ebenfalls kohäsiv haftend ausgebildeten äußeren Binde bereitgestellt.

Kohäsive Haftung bedeutet, dass keine Anhaftung an z. B. Haut oder Bekleidung erfolgt, sondern die Haftung nur zwischen den Bindenlagen (Oberfläche zu Oberfläche) besteht.

Die Bestimmung der Haftkräfte erfolgt dabei nach dem nachfolgend beschriebenen Verfahren:
Die Haftkraft ist die ermittelte Kraft, die für das Trennen von kohäsiven Proben im sog. 180 ° T-Peel- Test benötigt wird.

Das kohäsive beschichtete Textil wird spannungslos und faltenfrei ausgelegt. Daraus wird eine 10cm breite und 40cm lange Probe geschnitten. Der 40cm lange Probestreifen wird in der Mitte in 2 Streifen der Länge 20 cm durchgeschnitten.

Die beiden 20cm langen Streifen werden so aufeinander gelegt, dass die Seite A des ersten Streifens auf der Seite B des zweiten Streifens liegt. Die so vorbereitete Probe wird auf eine beheizte Edelstahlplatte (40 °C) gelegt und mit einer beheizten Metallwalze (40 °C) insgesamt 40-mal innerhalb von 30 sec gewalzt (20x hin und zurück).

Das Gewicht der Metallwalze beträgt 8 kg für 10 cm Probenbreite, d.h. 0,8 kg je cm Probenbreite für andere Breiten als 10 cm.

Anschließend wird die Kraft zur Trennung der Lagen der Probe in einem Kraft-Dehnungs-Prüfgerät (Hersteller: ZWICK, INSTRON) ermittelt. Dazu wird das Ende der ersten Lage in der unteren Klemme und das andere Ende der zweiten Lage in der oberen Klemme eingespannt, wobei darauf geachtet wird, dass die Probe möglichst ohne Verzug, d.h. spannungsarm, zwischen den Klemmen positioniert ist, damit keine "Ruhekraft" anliegt. Diese Anordnung entspricht einem 180° T-Peel- Test. Zur Messung bewegen sich die Klemmen vertikal auseinander, wobei die dabei wirkende Trennkraft (entspricht der Momentan- Haftkraft der Probe) kontinuierlich registriert wird. Durch Integration der Kraft über den zurückgelegten Weg der Klemmen wird die Trennarbeit und daraus die mittlere Trennkraft = Haftkraft in cN/cm erfasst und berechnet. Diese Haftkraft entspricht den Zahlenangaben bei den Beispielen.

Dabei ist es besonders vorteilhaft, wenn die Haftkraft des kohäsiv haftenden Bindenabschnitts sowie der kohäsiv haftenden zweiten Binde 20-150 cN/cm, weiter bevorzugt 30-100 cN/cm und weiter bevorzugt 40-80 cN/cm beträgt.

Vorzugsweise sind eine oder beide Binden auf einer Seite mit einem Haftklebemittel offenporig beschichtet, so dass von dieser Oberfläche eine kohäsive Haftfunktion ausgeht. Bei der Kompressionsbinde (äußere Binde) ist auch eine beidseitig kohäsive Beschichtung denkbar.

Die Komponenten (Binden) haften im angelegten Zustand aufeinander und weisen eine synergistische Kompressionswirkung auf. Darunter wird das Zusammenwirken des Kompressionsdruckes gemeinsam auf dem Körperteil verstanden. Darüber hinaus soll der Mehrlagenverband das Problem der leichten ambulatorischen Hypertonie in Venen sowie Arterien während des Tragens als Dauerverband lösen.

Zur Herstellung der inneren Binde dient beispielhaft ein Verfahren zur Herstellung einer vliesbasierten Kompressionsbinde umfassend eine Thermobondvliesschicht, die mit einer superhydrophilen oder superhydrophoben Vliesschicht vernäht wird. Dabei wird das Thermobondvlies einer Kettenwirkmaschine in nicht gedehntem Zustand zusammen mit einem Wasserstrahlvlies, welches auch schon vorvernadelt sein kann, zugeführt. In der Kettenwirkmaschine werden das Vlies (Thermobond) und das superhydrophile oder superhydrophobe Vlies mit einem elastischen Material so vernäht, dass ein von Hand nicht mehr trennbarer und trotzdem ein kontrollierbar elastischer Verbund entsteht. Das aus dem fertig vernähten Flächengebilde entstehende Polstermaterial weist immer auch eine optimierte Dehnung auf.

Eine optimierte Dehnung ist im Allgemeinen das Resultat der Einarbeitung von elastischen Fäden (hochgedrehte Baumwollfäden als Spinn- oder Zwirnkreppfäden, texturierte Polyamid- oder Polyestergarne, Gummi- bzw. PolyurethanElastanfäden) in ein unelastisches Vlies, z. B. ein steifes Thermobond-Vlies. Die Nähtechnik des Vlieses mit elastischem Material ist durch die Stichlänge und Spannung so reguliert, dass die Fasern auf einer Seite des Verbunds eine Ausgleichschicht (Polsterschicht) bildet und so einen hohen Hautkomfort aufweisen, so dass die innere Binde an sich zwei erkennbar unterschiedliche Seiten hat, die für den Druckausgleich sehr funktional sind. Diese Lage ist identisch mit der in DE102015226706 offenbarten Polsterlage.

Die Erfindung betrifft insbesondere (Komponente B) ein Verfahren zur Herstellung einer erfindungsgemäßen vliesbasierten Kompressionsbinde umfassend beispielhaft eine autogen verbundene Faserfläche, die durch einen chemischen Wirkstoff oder in einem thermischen oder mechanischen Verfahren verfestigt wird, genannt Thermobondvliesschicht, und einem elastischen, nicht-elastomerischen Garn. Dabei wird die Thermobondvliesschicht mit kontrollierter Zugkraft in einer Kettenwirkmaschine durch nicht-elastomerische hitzeschrumpffähige Textilfäden in kontrolliert gedehntem Zustand vernäht. Das aus dem fertig vernähten Flächengebilde entstehende kompressionsfähiges Material weist immer eine optimierte Dehnung auf; die optimierte Dehnung ist das Resultat der Einarbeitung von nicht-elastomerischen Fäden (texturierte Polyamid- oder Polyestergarne) und einem unelastischen Vlies, z. B. starren Thermobond-Vlies. Die Nähtechnik des Vlieses mit nicht-elastomerischen Materialien und die Stichlänge sind so reguliert, dass ein limitierter Kompressionsdruck von 10 bis 30 mm Hg in der späteren Anwendung sicher erzielt wird.

Das vernähte Flächengebilde kann dann mittels Längskonfektionierung zu Binden weiterverarbeitet werden. Diese Binde kann als Hilfsmittel in der Kompressionstherapie eingesetzt werden, vorzugsweise als zweite (äußere) Lage eines 2-Layer-Kompressionsverbandes.

Die Kompressionsbinde hat vorzugsweise zwei erkennbare und unterschiedliche Seiten.

Als zweite (äußerer) Layer reguliert die Binde, den gesamten Anpressdruck und sorgt für die notwendige Steifigkeit. Durch die nicht-elastomerischen und elastichen Fäden wird erreicht, dass eine erfindungsgemäße Kompressionsbindenzusammenstellung ein Druck von 20- 40 mm Hg nicht unter- und überschreitet. Diese Funktion wird nicht von Konkurrenzprodukten erzeugt.

Die Erfindung wird im Folgenden anhand einer Zeichnung näher beschrieben. Weitere Vorteile und Merkmale der Erfindung ergeben sich darüber hinaus aus den übrigen Anmeldungsunterlagen.

In der Zeichnung zeigt:
Figur 1 ein Herstellverfahren einer erfindungsgemäßen Kompressionsbinde und
Figur 2 ein Herstellungsverfahren für eine erste Binde einer Kompressionsbindenzusammenstellung gemäß der Erfindung.

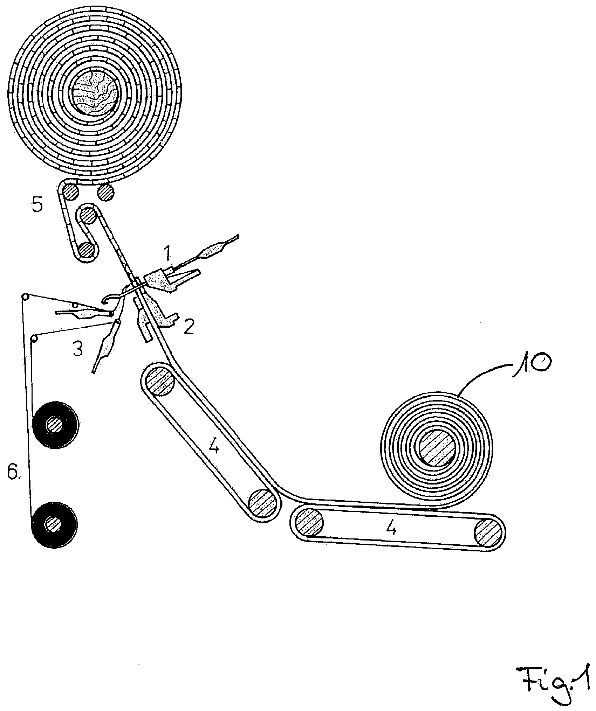

Figur 1 zeigt ein Herstellungsverfahren für eine erfindungsgemäße Kompressionsbinde, wie sie auch als äußere Binde für eine erfindungsgemäße Kompressionsbindenzusammenstellung dienen kann. Hierbei ist mit dem Bezugszeichen 10 ein zuzuführendes Vliesmaterial, insbesondere ein Thermobond-Vliesmaterial bezeichnet, das über Fördereinrichtungen 4 einer Nähwirkeinrichtung zugeführt wird. Mit den Bezugszeichen 1, 2 und 3 ist die Nähwirkeinrichtung bezeichnet, bei der es sich um eine herkömmliche Nähwirkeinrichtung zur Vornahme eines Übernähens mittels des Maliwat- oder Malimo -Verfahrens handelt. Hierzu werden elastische Nähfäden über die Rollen, die mit 6 bezeichnet sind, zugeführt. Das übernähte und damit elastifizierte Vlies wird beim Bezugszeichen 5 aufgewickelt. Im Anschluss daran kann dann eine Konfektionierung zu einer erfindungsgemäßen Kompressionsbinde vorgenommen werden.

Ein erstes bevorzugtes Ausführungsbeispiel ist in der nachfolgenden Tabelle dargestellt:

Ausführungsbeispiel Kompressionsbinde:

| | |
|---|---|
| Grund-Vlies | Faservlies (Stapelfaservlies) aus Baumwolle, Wolle, Viskose, Polyamid, Polyester, Acryl, Polyolefin oder Mischungen dieser Faserstoffe (mechanisch oder chemisch gebunden) |
| | Oder Spunbond aus Polyamid, Polyester, Polyolefin, Acryl oder Mischungen davon |
| Flächengewicht/Farbe Grund-Vlies | 20 - 40 g/m², weiß oder bunt |
| Struktur Grund-Vlies | Glatt oder offenporig oder perforiert oder geprägt |
| Nähfaden | 78 dtex bis 320 dtex texturiertes |
| | Multifilamentgarn aus Polyamid 6, 6.6, Polyester, Poly-ethylenterephthalat, Poly-butylenterephthalt |
| Farbe Nähfaden | Weiß, farblos/transparent oder bunt |
| Nähfadendichte | 24 bis 96 Fäden je 10 cm Breite |
| Nähfaden-Stichlänge | 2,0 bis 5,0 mm |
| Nähfaden-Bindung | Offene Franse oder geschlossene Franse oder Trikot |
| Flächengewicht Komponente B (DIN 61632) | Unbeschichtet: 25 bis 80 g/m² gedehnt |
| | Beschichtet: 30 bis 100 g/m² gedehnt |
| Dehnbarkeit (DIN 61632 bei K = 3 N/cm) | 40 bis 100% in Längsrichtung (MD) |
| | 0 bis 50% in Querrichtung (CD) |

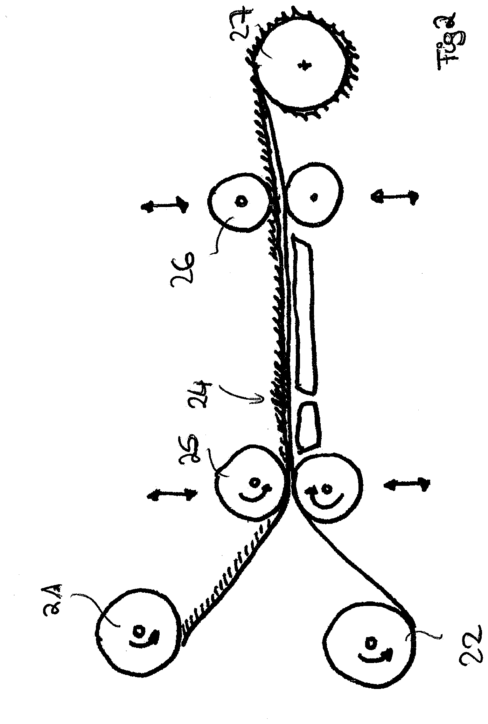

In Figur 2 ist dargestellt, wie zum einen eine Polsterschicht 21 aus einem Wattevlies und einer Stützschicht 22 hier als Thermobondvliesschicht aus einem Rollenmaterial zugeführt werden und in einem Nähwirkverfahren mittels eine Kettenwirkmaschine, die mit dem Bezugszeichen 24 versehen ist, miteinander verbunden werden. Dabei werden zuvor über eine Rollenführung 25 die beiden Lagen aufeinander fixiert. Das Nähwirkverfahren arbeitet dabei mit einem Haken, mittels dem die Schichten mit dem elastischen Nähfäden übernäht werden. Das miteinander über den elastischen Nähfaden verbundene Material wird dann durch eine weitere Rollenführung 26 geführt und auf eine Rolle 27 aufgewickelt, wobei gegebenenfalls vorab eine Konfektionierung in Längsrichtung zu Binden erfolgen kann. Bei dem Nähfaden handelt es sich um einen elastisch vorgedehnten Faden, der mit einer vorgegebenen Stichlänge und einer vorgegebenen Nähfadenspannung eingebracht wird, und so nach Entspannung des elastischen Materials zu einem Zusammenziehen des Lagenverbundes (Flächengebildes) führt.

## Patentansprüche

1. Kompressionsbinde umfassend eine vliesbasierte Bindenlage, die durch elastische Textilfäden mittels Nähwirkverfahren übernäht ist, **dadurch gekennzeichnet, dass** die Textilfäden hitzeschrumpffähig und nicht elastomer sind und die Kompressionsbinde eine elastische Dehnbarkeit nach Hitzeschrumpfung der Textilfäden um 50-200%, insbesondere von 50 % bis 90 % und insbesondere 50 % bis 70 % aufweist.

2. Kompressionsbinde nach Anspruch 1, **dadurch gekennzeichnet, dass** die Bindenlage aus einem chemisch, thermisch und/oder mechanisch verfestigten Vliesstoff besteht und insbesondere ein Thermobondvlies ist.

3. Kompressionsbinde nach Anspruch 1 oder 2, **dadurch gekennzeichnet, dass** der Vliesstoff unelastisch oder dehnungselastisch ist.

4. Kompressionsbinde nach einem der vorstehenden Ansprüche, **dadurch gekennzeichnet, dass** die Textilfäden texturierte Polyamidgarne und/oder texturierte Polyestergarne und/oder Polyethylenepthalat und/oder Polybutylenterephthalt oder Kombinationen hiervon sind.

5. Kompressionsbinde nach einem der vorstehenden Ansprüche, **dadurch gekennzeichnet, dass** die Bindenlage aus einem Faservlies umfassend Baumwolle, Wolle, Viskose, Polyamid, Polyester, Acryl, Polyolefin oder Mischungen dieser Faserstoffe gebildet ist und insbesondere hieraus besteht und insbesondere mechanisch oder chemisch gebunden ist oder dass die Bindenlage ein Spunbond aus Polyamid, Polyester, Polyolefin, Acryl oder Mischungen umfasst und insbesondere daraus besteht.

6. Kompressionsbinde nach einem der vorangehenden Ansprüche, **dadurch gekennzeichnet, dass** die Stichlänge 2 bis 5 mm des Nähwirkverfahrens pro Umdrehung beträgt bei einer Nähfadenspannung von höchstens 10 cN.

7. Kompressionsbinde nach einem der vorangehenden Ansprüche, **dadurch gekennzeichnet, dass** Dehnbarkeit der Kompressionsbinde gemessen nach DIN 61632 von 40 bis 100% in Längsrichtung (MD) beträgt.

8. Kompressionsbindenzusammenstellung umfassend eine erste innere Binde (12) sowie eine zweite äußere Binde, wobei die zweite äußere Binde im Gebrauch über die erste innere Binde (12) anlegbar ist, **dadurch gekennzeichnet, dass** die erste innere Binde einen ersten Abschnitt (L1) umfasst, der zumindest auf seiner der Haut eines Trägers zugewandten Seite eine Polsterschicht aufweist und einen kohäsiv haftenden zweiten Abschnitt (L2) aufweist und wobei die zweite äußere Binde eine Kompressionsbinde nach einem der Ansprüche 1 bis 7 ist.

9. Kompressionsbindenzusammenstellung nach Anspruch 8, **dadurch gekennzeichnet, dass** die beiden Abschnitte (L1, L2) der ersten inneren Binde (12) miteinander verbunden sind und in Längsrichtung der Binde (12) aneinander anschließen oder einander ganz oder teilweise überdecken.

10. Kompressionsbindenzusammenstellung nach einem der Ansprüche 8 oder 9, **dadurch gekennzeichnet, dass** die erste Binde eine erste Polsterschicht (1) und eine zweite Stützschicht (2) umfasst und die beiden Schichten (1,2) im ungedehnten Zustand mittels Nähwirkverfahren über einen elastischen Nähfaden miteinander verbunden sind und die Stichläge 1,5 bis 3 mm/U beträgt bei einer Nähfadenspannung von höchstens 4 cN.

11. Kompressionsbindenzusammenstellung nach einem der vorangehenden Ansprüche 8 bis 10, **dadurch gekennzeichnet, dass** das Material einer oder beider der Schichten (21, 22) der inneren Binde unelastisch ist.

12. Kompressionsbindenzusammenstellung nach einem der vorangehenden Ansprüche 8 bis 11, **dadurch gekennzeichnet, dass** mindestens eine der beiden Schichten (21,22) aus Polsterschicht (21) und Stützschicht (22) aus einem Vliesmaterial bestehen und/oder wobei insbesondere die Polsterschicht (21) ein Thermofusionsvlies ist.

13. Kompressionsbindenzusammenstellung nach einem der vorangehenden Ansprüche 8 bis 12, **dadurch gekennzeichnet, dass** die erste und die zweite Binde sich vollflächig und insbesondere kantengenau überdecken und über den gesamten Dehnungsbereich 0 bis Dfix haftend miteinander verbunden sind.

14. Verfahren zum Herstellen einer Kompressionsbinde nach einem der Ansprüche 1 bis 7, **dadurch gekennzeichnet, dass** eine vliesbasierte Bindenlage mittels elastischen Textilfäden in einem Nähwirkverfahren übernäht wird, wobei die übernähte Bindenlage in einem weiteren Schritt einer Hitzebehandlung unterzogen wird, und die Textilfäden hierbei eine Schrumpfung zwischen 50 und 200% insbesondere zwischen 50% und 70% erfahren.

15. Kompressionsbinde nach einem der Ansprüche 1 bis 7 zur Verwendung bei der Behandlung von Venenerkrankungen, chronischer venöser Insuffizienz, Ulcus curis venosum auch bei begleitender peripherer arterieller Verschlusskrankheit.

## Claims

1. Compression bandage comprising a non-woven-based bandage layer which is overstitched with elastic textile threads by means of a stitch-bonding process, **characterized in that** the textile threads are heat-shrinkable and not elastomeric and the compression bandage has, after heat shrinkage of the textile threads, an elastic stretchability of 50-200%, in particular from 50% to 90% and in particular 50% to 70%.

2. Compression bandage according to claim 1, **characterized in that** the bandage layer consists of a chemically, thermally and/or mechanically consolidated non-woven fabric and is in particular a thermobonded non-woven.

3. Compression bandage according to claim 1 or claim 2, **characterized in that** the non-woven fabric is inelastic or stretch-elastic.

4. Compression bandage according to any of the preceding claims, **characterized in that** the textile threads are textured polyamide yarns and/or textured polyester yarns and/or polyethylene phthalate and/or polybutylene terephthalate or combinations thereof.

5. Compression bandage according to any of the preceding claims, **characterized in that** the bandage layer is formed from a fibrous non-woven comprising cotton, wool, viscose, polyamide, polyester, acrylic, polyolefin or mixtures of these fibers and in particular consists thereof and is in particular mechanically or chemically bonded or **in that** the bandage layer comprises a spin-bonded fabric made of polyamide, polyester, polyolefin, acrylic or mixtures and in particular consists thereof.

6. Compression bandage according to any of the preceding claims, **characterized in that** the stitch length of the stitch-bonding process is 2 to 5 mm per revolution using a stitching thread tension of at most 10 cN.

7. Compression bandage according to any of the preceding claims, **characterized in that** the stretchability of the compression bandage, measured according to DIN 61632, is from 40 to 100% in the longitudinal direction (MD).

8. Compression bandage assembly comprising a first inner bandage (12) and a second outer bandage, it being possible to apply the second outer bandage, during use, over the first inner bandage (12),**characterized in that** the first inner bandage comprises a first portion (L1), which has a cushioning layer at least on its side facing the skin of a wearer, and a cohesively adhering second portion (L2), and the second outer bandage being a compression bandage according to any of claims 1 to 7.

9. Compression bandage assembly according to claim 8, **characterized in that** the two portions (L1, L2) of the first inner bandage (12) are connected to one another and adjoin one another in the longitudinal direction of the bandage (12) or completely or partially overlap one another.

10. Compression bandage assembly according to either of claims 8 or 9, **characterized in that** the first bandage comprises a first cushioning layer (1) and a second supporting layer (2), and the two layers (1,2) are connected to one another in the unstretched state via an elastic stitching thread by means of a stitch-bonding process and the stitch length is 1.5 to 3 mm/rev using a stitching thread tension of at most 4 cN.

11. Compression bandage assembly according to any of the preceding claims 8 to 10, **characterized in that** the material of one or both of the layers (21, 22) of the inner bandage is inelastic.

12. Compression bandage assembly according to any of the preceding claims 8 to 11, **characterized in that** at least one of the two layers (21,22) consisting of the cushioning layer (21) and the supporting layer (22) consists of a non-woven material and/or in particular the cushioning layer (21) being a thermofusion non-woven.

13. Compression bandage assembly according to any of the preceding claims 8 to 12, **characterized in that** the first and the second bandage overlap one another completely and particularly with their edges aligned and are adhesively connected to one another over the entire stretch range 0 to Dfix.

14. Method for producing a compression bandage according to any of claims 1 to 7, **characterized in that** a non-woven-based bandage layer is overstitched by means of elastic textile threads in a stitch-bonding process, the overstitched bandage layer being subjected to heat treatment in a further step, during which heat treatment the textile threads experience a shrinkage of between 50 and 200%, in particular between 50% and 70%.

15. Compression bandage according to any of claims 1 to 7 for use in the treatment of venous diseases, chronic venous insufficiency, ulcus curis venosum, also in the case of accompanying peripheral arterial occlusive disease.

## Revendications

1. Bande de compression comprenant une épaisseur de bande à base de non-tissé qui est surpiquée par des fibres textiles élastiques au moyen d'un procédé de couture-tricotage, **caractérisée en ce que** les fibres textiles sont thermorétractables et non élastomères et la bande de compression présente une extensibilité élastique après thermorétraction des fibres textiles d'environ 50 à 200 %, en particulier de 50 % à 90 % et en particulier de 50 % à 70 %.

2. Bande de compression selon la revendication 1, **caractérisée en ce que** l'épaisseur de bande est constituée d'une matière non tissée consolidée chimiquement, thermiquement et/ou mécaniquement et est en particulier un non-tissé thermolié.

3. Bande de compression selon la revendication 1 ou 2, **caractérisée en ce que** la matière non tissée est non élastique ou élastique à l'extension.

4. Bande de compression selon l'une des revendications précédentes, **caractérisée en ce que** les fibres textiles sont des fils de polyamide texturés et/ou fils de polyester texturés et/ou polyéthylène phtalate et/ou polybutylène téréphtalate ou combinaisons de ceux-ci.

5. Bande de compression selon l'une des revendications précédentes, **caractérisée en ce que** l'épaisseur de bande est formée à partir d'un non-tissé comprenant coton, laine, viscose, polyamide, polyester, acrylique, polyoléfine ou des mélanges de ces matières fibreuses et est en particulier constituée de celui-ci, et en particulier est reliée mécaniquement ou chimiquement, ou **en ce que** l'épaisseur de bande comprend un spunbond en polyamide, polyester, polyoléfine, acrylique ou des mélanges, et en particulier est constituée de celui-ci.

6. Bande de compression selon l'une des revendications précédentes, **caractérisée en ce que** la longueur de point de couture du procédé de couture-tricotage est de 2 à 5 mm par tour pour une tension de fibre de couture de 10 cN au maximum.

7. Bande de compression selon l'une des revendications précédentes, **caractérisée en ce que** l'extensibilité de la bande de compression, mesurée selon la norme DIN 61632, est de 40 à 100 % dans le sens de la longueur (MD).

8. Ensemble de bandes de compression comprenant une première bande intérieure (12) ainsi qu'une seconde bande extérieure, dans lequel la seconde bande extérieure peut être appliquée au-dessus de la première bande intérieure (12) lors de l'utilisation, **caractérisé en ce que** la première bande intérieure comprend une première section (L1) qui présente une couche de rembourrage au moins sur son côté faisant face à la peau d'un porteur et qui présente une seconde section (L2) adhérant de manière cohésive, et dans lequel la seconde bande extérieure est un bande de compression selon l'une des revendications 1 à 7.

9. Ensemble de bandes de compression selon la revendication 8, **caractérisé en ce que** les deux sections (L1, L2) de la première bande intérieure (12) sont reliées l'une à l'autre et se rejoignent dans le sens de la longueur de la bande (12) ou se recouvrent totalement ou partiellement.

10. Ensemble de bandes de compression selon l'une des revendications 8 ou 9, **caractérisé en ce que** la première bande comprend une première couche de rembourrage (1) et une seconde couche de soutien (2) et les deux couches (1, 2) sont reliées l'une à l'autre à l'état non étendu au moyen d'un procédé de couture-tricotage par l'intermédiaire d'une fibre de couture élastique et la longueur de point de couture est de 1,5 à 3 mm/tr pour une tension de fibre de couture de 4 cN au maximum.

11. Ensemble de bandes de compression selon l'une des revendications précédentes 8 à 10, **caractérisé en ce que** le matériau de l'une ou des deux couches (21, 22) de la bande intérieure est non élastique.

12. Ensemble de bandes de compression selon l'une des revendications précédentes 8 à 11, **caractérisé en ce qu'**au moins l'une des deux couches (21, 22) est constituée de la couche de rembourrage (21) et la couche de soutien (22) est constituée d'un matériau non tissé et/ou dans lequel, en particulier, la couche de rembourrage (21) est un non-tissé de thermofusion.

13. Ensemble de bandes de compression selon l'une des revendications précédentes 8 à 12, **caractérisé en ce que** la première et la seconde bande se recouvrent sur toute leur surface et en particulier sur les bords, et sont reliées entre elles par adhérence sur toute la plage d'extension 0 à Dfix.

14. Procédé de fabrication d'une bande de compression selon l'une des revendications 1 à 7, **caractérisé en ce qu'**une épaisseur de bande à base de non-tissé est surpiquée au moyen de fibres textiles élastiques dans un procédé de couture-tricotage, dans lequel l'épaisseur de bande surpiquée est soumise dans une étape supplémentaire à un traitement thermique, et les fibres textiles subissent alors un rétrécissement compris entre 50 et 200 %, en particulier entre 50 % et 70 %.

15. Bande de compression selon l'une des revendications 1 à 7, destinée à être utilisée dans le traitement des maladies veineuses, de l'insuffisance veineuse chronique, de l'ulcère variqueux, même en présence d'une maladie obstructive de l'artère périphérique concomitante.
